(19) Europäisches Patentamt
European Patent Office
Office européen des brevets

(11) Veröffentlichungsnummer: **0 249 844**
**A1**

# (12) EUROPÄISCHE PATENTANMELDUNG

(21) Anmeldenummer: **87108241.8**

(51) Int. Cl.$^4$: **C07D 307/935**

(22) Anmeldetag: **06.06.87**

(30) Priorität: **18.06.86 DE 3620442**

(43) Veröffentlichungstag der Anmeldung:
**23.12.87 Patentblatt 87/52**

(84) Benannte Vertragsstaaten:
**AT CH DE ES FR GB IT LI SE**

(71) Anmelder: **Merck Patent Gesellschaft mit beschränkter Haftung**
**Frankfurter Strasse 250**
**D-6100 Darmstadt(DE)**

(72) Erfinder: **Riefling, Bernhard, Dr.**
**Zöllerstrasse 28**
**D-6100 Darmstadt(DE)**

(54) **Ketolactone.**

(57) Neue Ketolactone der Formel I

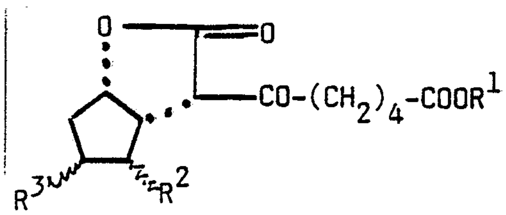

I

worin

$R^1$ H, Alkyl mit 1-6 C-Atomen oder Aryl mit 6-14 C-Atomen und

$R^2$ und $R^3$ zusammen ein O-Atom oder auch zusammen eine Bindung

bedeuten,

sowie deren Salze können als Zwischenprodukte bei der Herstellung von Prostaglandinderivaten verwendet werden.

EP 0 249 844 A1

## Ketolactone

Die Erfindung betrifft neue Ketolactone der Formel I

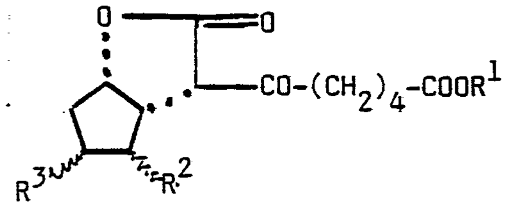

I

worin

$R^1$ H, Alkyl mit 1-6 C-Atomen oder Aryl mit 6-14 C-Atomen und

$R^2$ und $R^3$ zusammen ein O-Atom oder auch zusammen eine Bindung

bedeuten,

sowie deren Salze,

sowie ein Verfahren zu ihrer Herstellung, dadurch gekennzeichnet, daß man ein Lacton der Formel II

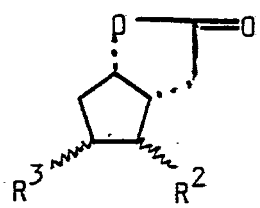

II

worin

$R^2$ und $R^3$ die angegebene Bedeutung haben,

mit einem Adipinsäurehalbesterhalogenid der Formel III

$Hal-CO-(CH_2)_4-COOR^4$ III

worin

$R^4$ Alkyl mit 1-6 C-Atomen oder Aryl mit 6-14 C-Atomen und

Hal Cl oder Br

bedeuten,

umsetzt,

und/oder daß man eine Verbindung der Formel I ($R^2$ + $R^3$ = Bindung) durch Behandeln mit einem Oxydationsmittel in eine Verbindung der Formel I ($R^2$ + $R^3$ = O) umwandelt und/oder eine Verbindung der Formel I ($R^1$ = $R^4$) zu einer Verbindung der Formel I ($R^1$ = H) verseift und/oder eine Verbindung der Formel I ($R^1$ = H) verestert oder durch Behandeln mit einer Base in eines ihrer Salze überführt.

Die Verbindungen der Formel I können als Zwischenprodukte bei der Herstellung von Prostaglandinderivaten verwendet werden. Insbesondere können durch Behandlung mit Basen, z. B. mit BaO in wasserhaltigem Medium, unter Verseifung, Aufspaltung des Lactonrings und Decarboxylierung 6-Oxo-7-(2-$R^2$-3-$R^3$-5α-hydroxy-cyclopentyl)-heptansäuren (IV) erhalten werden, deren Überführung in bestimmte 6-Oxo-13-thiaprostansäurederivate in der DE-OS 3401542 beschrieben ist.

Die bisher bekannte Synthese von IV zeigt erhebliche Nachteile. So verläuft sie über eine große Anzahl von Stufen, bei denen instabile Zwischenprodukte auftreten. Eine dieser Stufen ist eine Reduktion mit Diisobutylaluminiumlhydrid, einem teuren und im technischen Maßstab schwierig zu handhabenden Reagenz.

Der Erfindung lag die Aufgabe zugrunde, ein neues Verfahren und neue Zwischenprodukte zur Herstellung von IV und verwandten Verbindungen und damit zur Herstellung von Prostaglandinderivaten aufzufinden, die die Nachteile des bekannten Verfahrens nicht oder nur in geringerem Maße aufweisen und insbesondere für den großtechnischen Maßstab geeignet sind.

Diese Aufgabe wurde durch die Bereitstellung des neuen Verfahrens und der neuen Zwischenprodukte der Formel I gelöst.

Die über die Ketolactone I verlaufende neue Synthese von IV hat weniger Stufen als die bisher bekannte, verläuft über stabilere Zwischenprodukte und erlaubt die Verwendung billigerer Reagenzien.

In den Verbindungen der Formeln I und III bedeutet $R^1$ vorzugsweise H, Methyl oder Ethyl, $R^4$ vorzugsweise Methyl oder Ethyl. Weiterhin bedeuten beide Reste bevorzugt Propyl, Isopropyl, Butyl, Isobutyl, sek.-Butyl, tert.-Butyl, Pentyl, Isopentyl, Hexyl, Isohexyl, Phenyl, o-, m-oder p-Tolyl, o-, m-oder p-Fluorphenyl, o-, m-oder p-Chlorphenyl, o-, m-oder p-Methoxyphenyl, o-, m-oder p-Acetamidophenyl, o-, m-oder insbesondere p-Benzamidophenyl.

Die Ausgangsstoffe der Formeln II und III sind weitgehend bekannt. Sofern sie nicht bekannt sind, können sie in Analogie zu bekannten Verbindungen nach bekannten Methoden hergestellt werden.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II und III können gewünschtenfalls auch in situ gebildet werden, derart, daß man sie aus dem Reaktionsgemisch nicht isoliert, sondern sofort weiter zu den Verbindungen der Formel I umsetzt.

Vor der Umsetzung mit III wird das Lacton II zweckmäßig durch Behandeln mit einer Base in einem inerten Lösungsmittel in das entsprechende Anion übergeführt. Als Basen eignen sich z. B. Organometallverbindungen wie Butyllithium oder Alkalimetallamide wie $NaNH_2$, $KNH_2$ oder ein Bis-(trimethylsilyl)-alkalimetallamid, als Lösungsmittel z. B. Ether wie Tetrahydrofuran (THF), Diethylether, Diisopropylether, Methyl-tert.-butylether, Dioxan; die Reaktionstemperaturen liegen zweckmäßig zwischen etwa -40 und +20°, vorzugsweise zwischen -30 und +10°. Zu der erhaltenen Lösung wird anschließend zweckmäßig eine Lösung von III in einem inerten Lösungsmittel, z. B. einem der genannten Lösungsmittel zugegeben, wobei die Reaktionstemperaturen vorzugsweise zwischen -80 und +30° liegen.

Ein Cyclopentenderivat I ($R^2$ + $R^3$ = Bindung) kann, falls erwünscht, zum entsprechenden Epoxid I ($R^2$ + $R^3$ = O) oxydiert werden. Als Oxydationsmittel eignen sich insbesondere Peroxide und Hydroperoxide wie Peressigsäure, m-Chlorperbenzoesäure oder tert.-Butylhydroperoxid. Hydroperoxide werden zweckmäßig in Gegenwart eines Katalysators verwendet, z. B. eines Schwermetallkatalysators wie Molybdänhexacarbonyl, Vanadin(IV)oxid oder einem ihrer Derivate, z. B. Vanadin(IV)oxid-acetyl-acetonat. Die Oxydation erfolgt zweckmäßig in Gegenwart eines inerten Lösungsmittels, z. B. einer Carbonsäure wie Essigsäure, eines halogenierten Kohlenwasserstoffs wie Dichlormethan, Chloroform, Tetrachlorkohlenstoff, Trichlorethylen, oder eines aromatischen Kohlenwasserstoffs wie Benzol oder Toluol, bei Temperaturen zwischen etwa 0 und etwa 40°, vorzugsweise zwischen 15 und 30°. Es ist auch möglich, mehrstufig zu oxydieren, z. B. durch Anlagerung von HOBr zum Bromhydrin und anschließende Dehydrobromierung zum Epoxid.

Ein Ester der Formel I ($R^1$ = $R^4$) kann zu der entsprechenden Säure I ($R^1$ = H) verseift werden, zweckmäßig unter den üblichen milden alkalischen oder sauren Bedingungen, z. B. mit wässerigen oder wässerig-alkoholischen Lösungen von NaOH oder KOH bei Temperaturen zwischen 0 und 40°. Dabei wird gleichzeitig der Lactonring geöffnet; er kann durch anschließendes kurzes Behandeln mit einer Säure, z. B. Salzsäure, wieder geschlossen werden. Unter kräftigeren Bedingungen, z. B. mit wässerigen oder wässerig-alkoholischen Lösungen oder Suspensionen von Alkali-oder Erdalkalimetallhydroxiden oder -oxiden wie BaO bei höheren Temperaturen, etwa zwischen 50 und 100°, wird zusätzlich decarboxyliert, und es entstehen die oben genannten Verbindungen IV.

Eine Säure der Formel I ($R^1$ = H) oder eines ihrer reaktionsfähigen Derivate kann, falls erwünscht, in üblicher Weise mit einem Alkohol oder Phenol der Formel $R^4$-OH oder einem seiner reaktionsfähigen Derivate verestert werden, z. B. in Gegenwart einer starken Säure wie HCl oder $H_2SO_4$ oder eines wasserabspaltenden Mittels wie Dicyclohexylcarbodiimid, wobei man zweckmäßig in einem Überschuß der Verbindung der Formel $R^4$-OH und/oder in Gegenwart eines inerten Lösungsmittels bei Temperaturen zwischen 0 und 40° arbeitet.

Eine Säure der Formel I ($R^1$ = H) kann durch Umsetzung mit einer Base in eines ihrer Metall-bzw. Ammoniumsalze übergeführt werden. Als Salze kommen insbesondere die Natrium-, Kalium-, Magnesium-, Calcium-und Ammoniumsalze in Betracht.

"Übliche Aufarbeitung" bedeutet: man gibt bei Bedarf Wasser hinzu, extrahiert mit Dichlormethan oder Ethylacetat, trennt ab, wäscht die organische Phase mit Wasser, trocknet über Natriumsulfat, dampft ein und reinigt das erhaltene Rohprodukt chromatographisch an Kieselgel.

Beispiel 1

Eine Lösung von 3,4 ml Diisopropylamin in 10 ml THF wird unter Stickstoff auf 0° gekühlt und mit 14,5 ml einer 1,5 molaren Butyllithiumlösung versetzt. Man rührt 30 Min. nach, kühlt dann auf -78° und tropft eine Lösung von 1,43 g 2-Oxa-IßH,5ßH-bicyclo[3,3,0]oct-6-en-3-on in 5 ml THF langsam zu. Nach weiteren 30 Min. wird diese Mischung in eine im Trockeneisbad gekühlte Lösung von 2,15 g 5-Chlorcarbonylvaleriansäuremethylester in 10 ml THF getropft. Man rührt 30 Min. nach und gießt dann 30 ml einer gesättigten wäßrigen Citronensäurelösung zu. Übliche Aufarbeitung ergibt 2-Oxa-4-(5-methoxycarbonylpentanoyl)-IßH,5ßH-bicyclo[3,3,0]oct-6-en-3-on (Ia) als gelbliches Öl. Massenspektrum: m/e = 266.

Beispiel 2

Zu einer -30° kalten Mischung von 20 ml 1-molarer Bis-(trimethylsilyl)-lithiumamid/THF-Lösung in 20 ml THF wird eine Lösung von 1,61 g 2-Oxa-6α,7α-epoxy-IßH,5ßH-bicyclo[3,3,0]octan-3-on in 10 ml THF getropft. Man rührt noch 30 Min., kühlt dann auf -78° und tropft eine Lösung von 2,15 g 5-Chlorcarbonylvaleriansäuremethylester in 5 ml THF zu. Nach weiteren 30 Min. wird nach Zugabe von 30 ml gesättigter Citronensäurelösung auf übliche Weise aufgearbeitet. Man erhält 2-Oxa-4-(5-methoxycarbonylpentanoyl)-6α,7α-epoxy-1ßH,5ßH-bicyclo[3,3,0]octan-3-on (Ib).

Beispiel 3

Eine Mischung aus 5,32 g Ia und 0,5 g Natriumacetat in 12 ml Essigsäure wird auf 12-15° gekühlt und tropfenweise mit 5,7 g 40%iger Peressigsäure versetzt. Man rührt 2 Tage bei 12-15°, verdünnt mit 20 ml Wasser und gibt unter Eiskühlung bei 10-15° 2 g Natriumsulfit in kleinen Portionen zu. Nach negativer KJ-Stärkepapier-Probe wird in üblicher Weise weiter aufgearbeitet. Man erhält Ib.

Beispiel 4

Ein Gemisch aus 1 g Ia und 10 ml 10%iger methanolischer KOH-Lösung wird 0,5 Std. bei 20° gerührt. Man säuert mit wässeriger HCl an, arbeitet wie üblich auf, löst das erhaltene rohe 2-Oxa-4-(5-carboxypentanoyl)-IßH,5ßH-bicyclo[3,3,0]oct-6-en-3-on in 10 ml Pyridin, versetzt mit 1 g p-Benzamidophenol, 1 g Dicyclohexylcarbodiimid, und 0,1 g p-Toluolsulfonsäure, läßt 1 Tag bei 20° stehen, filtriert, rührt das Filtrat in Wasser ein und filtriert das ausgefallene 2-Oxa-4-(5-p-benzamidophenoxycarbonylpentanoyl)-IßH,5ßH-bicyclo[3,3,0]oct-6-en-3-on ab.

Verwendungsbeispiel

Eine Lösung von 2,66 g Ia (Rohprodukt) in 10 ml Methanol und 10 ml Wasser wird mit 5 g Bariumoxid versetzt und 16 Std. unter Stickstoff auf dem Dampfbad belassen (pH ≥ 10). Man filtriert, wäscht mit Wasser nach, säuert das Filtrat mit Citronensäure auf pH 2-3 an und extrahiert mit Dichlormethan. Man läßt die organische Phase 16 Std. stehen und extrahiert sie mit $NaHCO_3$-Lösung. Die wässerige Phase wird mit Citronensäure auf pH 5 angesäuert, mit Ethylacetat extrahiert und wie üblich aufgearbeitet. Man erhält 6-Oxo-7-(5α-hydroxy-2-cyclopenten-1α-yl)-heptansäure. Massenspektrum: m/e $(-H_2O)$ = 208.

**Ansprüche**

1. Ketolactone der Formel I

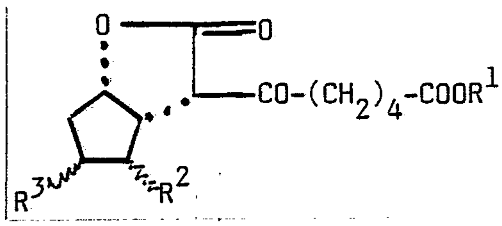

I

worin

$R^1$ H, Alkyl mit 1-6 C-Atomen oder Aryl mit 6-14 C-Atomen und

$R^2$ und $R^3$ zusammen ein O-Atom oder auch zusammen eine Bindung

bedeuten,

sowie deren Salze.

2.

a) 2-Oxa-4-(5-methoxycarbonyl-pentanoyl)-1ßH,5ßH-bicyclo[3,3,0]-oct-6-en-3-on.

b) 2-Oxa-6α,7α-epoxy-4-(5-methoxycarbonyl-pentanoyl),-IßH,5ßH-bicyclo[3,3,0]octan-3-on.

3. Verfahren zur Herstellung von Ketolactonen der Formel I sowie ihrer Salze, dadurch gekennzeichnet, daß man ein Lacton der Formel II

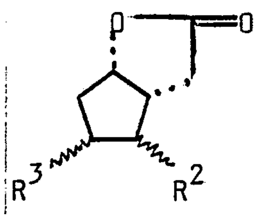

II

worin

$R^2$ und $R^3$ die angegebene Bedeutung haben,

mit einem Adipinsäurehalbesterhalogenid der Formel III

$Hal-CO-(CH_2)_4-COOR^4$ III

worin

$R^4$ Alkyl mit 1-6 C-Atomen oder Aryl mit 6-14 C-Atomen und

Hal Cl oder Br

bedeuten,

umsetzt,

und/oder daß man eine Verbindung der Formel I ($R^2$ + $R^3$ = Bindung) durch Behandeln mit einem Oxydationsmittel in eine Verbindung der Formel I ($R^2$ + $R^3$ = O) umwandelt und/oder eine Verbindung der Formel I ($R^1$ = $R^4$) zu einer Verbindung der Formel I ($R^1$ = H) verseift und/oder eine Verbindung der Formel I ($R^1$ = H) verestert oder durch Behandeln mit einer Base in eines ihrer Salze überführt.

4. Verwendung von Ketolactonen der Formel I bei der Synthese von Prostaglandinderivaten.

Europäisches Patentamt

# EUROPÄISCHER RECHERCHENBERICHT

Nummer der Anmeldung

EP 87108241.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 156 611 (ONO PHARMACEUTICAL CO., LTD.)<br>* Anspruch 1; Zusammenfassung *<br>-- | 1,4 | C 07 D 307/935 |
| A | US - A - 4 103 091 (MITSCHER)<br>* Verbindung II, 18 *<br>-- | 1,4 | |
| A | US - A - 3 823 138 (VERLAN)<br>* Zusammenfassung; Verbindung I *<br>-- | 1,4 | |
| A | US - A - 4 410 710 (BERKOWITZ)<br>* Verbindungen 6,7 *<br>-- | 1 | RECHERCHIERTE SACHGEBIETE (Int. Cl.4) |
| A | CHEMICAL ABSTRACTS, Band 89, Nr. 17, 23. Oktober 1978, Columbus, Ohio, USA<br>CAMPAIGNE, E.; BECKMAN, JEAN C. "A new synthesis of α-carboxy-γ-lactones and α-methylene-γ-lactones"<br>Seite 570, Spalte 2, Zusammenfassung-Nr. 146 472q<br>& Synthesis 1978, (5), 385-8<br>---- | 1 | C 07 D 307/00 |

Der vorliegende Recherchenbericht wurde für alle Patentansprüche erstellt.

| Recherchenort | Abschlußdatum der Recherche | Prüfer |
|---|---|---|
| WIEN | 20-08-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82